# EUROPÄISCHE PATENTSCHRIFT

(11) **EP 3 634 321 B1**
(45) Veröffentlichungstag und Bekanntmachung des Hinweises auf die Patenterteilung: **09.11.2022**
(21) Anmeldenummer: 18729945.8
(22) Anmeldetag: 06.06.2018
(51) Int. Cl.: A61F 2/66, A61F 2/68, A61F 2/70, A61F 2/74

(54) **ORTHOPÄDIETECHNISCHE GELENKEINRICHTUNG**
ORTHOPEDIC JOINT DEVICE
DISPOSITIF D'ARTICULATION ORTHOPÉDIQUE

(30) Priorität: 06.06.2017 DE 102017112457; 07.07.2017 DE 102017115267
(43) Veröffentlichungstag der Anmeldung: 15.04.2020
(73) Patentinhaber: Ottobock SE & Co. KGaA, 37115 Duderstadt (DE)
(72) Erfinder: KROLL-ORYWAHL, Olaf, 37154 Northeim (DE); MÜLLER, André, 37115 Duderstadt (DE); SIEWERT, Gordon, 37077 Göttingen (DE)
(74) Vertreter: Gramm, Lins & Partner Patent- und Rechtsanwälte PartGmbB
(86) Internationale Anmeldenummer: PCT/EP2018/064905
(87) Internationale Veröffentlichungsnummer: WO 2018/224552

(56) Entgegenhaltungen:
- WO-A1-2016/130150
- US-A1- 2006 069 448
- US-A1- 2016 158 031

## Beschreibung

Die Erfindung betrifft eine orthopädietechnische Gelenkeinrichtung mit einem Unterschenkelteil, einem Fußteil, das um eine Schwenkachse schwenkbar an dem Unterschenkelteil angeordnet ist, wenigstens einem ersten Energiespeicher und einem Koppelelement, das in eine Koppelstellung, in der ein Verschwenken des Fußteils relativ zu dem Unterschenkelteil um die Schwenkachse in einer Plantarflexionsrichtung zu einer Erhöhung der in den ersten Energiespeicher gespeicherten Energiemenge führt, und in eine Entkoppelstellung bringbar ist, wie in den Ansprüchen definiert.

Orthopädietechnische Gelenkeinrichtungen sind aus dem Stand der Technik seit langer Zeit bekannt. Sie können als Prothese oder Orthese ausgebildet sein und beispielsweise eine Knöchel-Fuß-Orthese (ankle foot orthosis AFO), als Knie-Knöchel-Fuß-Orthese (knee ankle foot orthosis KAFO) oder als Hüfte-Knie-Knöchel-Fuß-Orthese (hip knee ankle foot orthosis HKAFO) ausgebildet sein. Insbesondere kann die orthopädietechnische Gelenkeinrichtung eine Fußheberorthese sein. Diese werden insbesondere bei Patienten mit einer Fußheberschwäche, die sich beispielsweise in Folge einer Nervenerkrankung wie z.B. Multiple Sklerose oder nach einem Schlaganfall entwickeln kann, eingesetzt. Eine Fußheberschwäche ist insbesondere dadurch gekennzeichnet, dass eine Bewegung des Fußes in Dorsalflexionsrichtung nur eingeschränkt oder gar nicht möglich ist. Eine Fußheberorthese wird daher insbesondere dazu eingesetzt, dieser Beeinträchtigung entgegenzuwirken und beispielweise ein physiologischeres Gangbild zu erzeugen oder aber die Verletzungs- und Stolpergefahr eines Patienten zu vermindern und so insgesamt zu einer erhöhten Lebensqualität beizutragen.

Hierzu hat es sich als vorteilhaft erwiesen, wenn zumindest ein Energiespeicher vorgesehen ist, in welchem im Verlauf eines Gangzyklus Energie gespeichert und aus diesem wieder freigesetzt werden kann. Die Energie wird beispielsweise verwendet um das Fußteil mit einer unterstützenden Kraft zu beaufschlagen, die den Träger beim Durchführen einer Plantarflexion oder Dorsalflexion unterstützt.

Unter einer Plantarflexionsbewegung ist eine Beugung des Fußes um die Sprunggelenksachse in Richtung der Fußsohle zu verstehen. Der Winkel zwischen Unterschenkel und Fuß in der Sagittalebene vergrößert sich folglich im Verlauf einer Plantarflexionsbewegung. Unter einer Dorsalflexionsbewegung ist die zur Plantarflexion entgegengesetzte Bewegung zu verstehen. Sie geht folglich mit einer Verkleinerung des Winkels zwischen Unterschenkel und Fuß in der Sagittalebene einher.

Der Gangzyklus eines Menschen kann allgemein in vier Phasen unterteilt werden. Die sogenannte kontrollierte Plantarflexionsphase, die von einem Aufsetzen der Ferse auf den Boden, dem sogenannten "heel strike", bis zu einem vollständigen Aufliegen des Fußes auf dem Boden verläuft. Die für diese Phase größte Plantarflexion, entsprechend dem größten in dieser Phase erreichten Winkel zwischen Unterschenkel und Fuß in der Sagittalebene, ist in dieser Phase dann erreicht, wenn der Fuß erstmals vollständig auf dem Boden aufliegt.

Hieran schließt sich die kontrollierte Dorsalflexionsphase an. Die kontrollierte Dorsalflexionsphase geht mit Erreichen der maximalen Dorsalflexion, also dem Erreichen des kleinsten Winkels zwischen Unterschenkel und Fuß in der Sagittalebene, in die angetriebene Plantarflexionsphase über. Dieser Übergang wird im Moment des Abhebens der Ferse vom Boden erreicht. Die angetriebene Plantarflexionsphase endet wiederum mit dem Verlust des Bodenkontaktes des Fußes, dem sogenannten "toe-off" und geht in die Schwungphase über. Die Schwungphase endet ihrerseits mit dem erneuten Aufsetzen der Ferse auf dem Boden, dem "heel strike".

Aus der US 8,696,764 B2, der US 2004/0064195 A1 und der US 2013/0046218 A1 sind bereits Fußheberorthesen bekannt, die eine Energiespeichereinrichtung aufweisen, in der zumindest teilweise während der kontrollierten Dorsalflexionsphase Energie gespeichert wird. Diese Energie wird verwendet, um die angetrieben Plantarflexion zu unterstützen. Daher wird sie in dieser Phase abgegeben.

Der Träger einer Fußheberorthese benötigt insbesondere in der angetriebenen Plantarflexionsphase und nach dem "toe-off" Unterstützung durch die Orthese, da hier Bewegungen des Fußes relativ zum Unterschenkel erforderlich sind, die durch den Patienten mit Muskelkraft hervorgerufen werden müssen.

Aus dem Stand der Technik sind Orthesen bekannt, die bei einer Plantarflexion Energie speichern. Diese Energie wird während der Schwungphase abgegeben um eine Dorsalflexionsbewegung des Fußes hervorzurufen und die Zehen anzuheben. Dadurch wird die Stolpergefahr verringert. Aus der WO 2016/130150 A1 und der US 8920517 B2 ist die Verwendung hydraulischer Energiespeicher bekannt.

Nachteilig ist insbesondere im Hinblick auf solche Energiespeicher, dass die in der Schwungphase abzugebende Energie in der angetriebenen Plantarflexionsphase gespeichert werden muss und daher durch Muskelkraft oder eine andere Energiespeichereinheit aufgebracht werden muss. Die ohnehin schwachen Muskeln des Patienten müssen folglich neben der Energie für die angetriebene Plantarflexion zusätzlich die zu speichernde Energie aufbringen, die in der Schwungphase abzugeben ist.

Aus dem Stand der Technik ist es daher bekannt, einen weiteren Energiespeicher vorzusehen, der eingerichtet ist, Energie während einer Dorsalflexion zu speichern, sodass diese während einer Plantarflexionsbewegung freigesetzt werden kann. In einer solchen Orthese wird der zweite Energiespeicher während der kontrollierten Dorsalflexionsphase aufgeladen und die Energie während der sich anschließenden angetriebenen Plantarflexionsphase wieder freigesetzt. Da während der angetriebenen Plantarflexionsphase der erste Energiespeicher geladen wird, arbeiten die beiden Energiespeicher zumindest teilweise "gegeneinander".

Die der Erfindung zugrundeliegende Aufgabe ist es daher, eine effizientere orthopädietechnische Gelenkeinrichtung vorzuschlagen, die die beschriebenen Nachteile aus dem Stand der Technik überwindet.

Die Erfindung löst die gestellte Aufgabe durch eine gattungsgemäße orthopädietechnische Gelenkeinrichtung gemäß dem Oberbegriff des Anspruchs 1, die sich dadurch auszeichnet, dass die orthopädietechnische Gelenkeinrichtung wenigstens ein Freigabeelement aufweist, das in eine Freigabestellung und in eine Sperrstellung bringbar ist, wobei die in dem ersten Energiespeicher gespeicherte Energiemenge freigebbar ist, indem das Freigabeelement in die Freigabestellung gebracht wird.

Unter einem Freigabeelement ist insbesondere ein Element zu verstehen, welches eine Freisetzung der in dem ersten Energiespeicher gespeicherten Energiemenge verhindert, wenn es sich in der Sperrstellung befindet. Erfindungsgemäß kann die innerhalb des ersten Energiespeichers gespeicherte Energie insbesondere zu einem beliebigen Zeitpunkt des Gangzyklus und kontrolliert freigegeben werden, indem das Freigabeelement in die Freigabestellung gebracht wird.

Durch die erfindungsgemäße Vorrichtung ist es insbesondere möglich, Energie in dem ersten Energiespeicher während der kontrollierten Plantarflexionsphase zu speichern und diese beispielsweise erst nach dem "toe-off" am Übergang zwischen der angetriebenen Plantarflexionsphase und der Schwungphase wieder freizugeben. Ein zwischenzeitliches Entladen und Laden des Energiespeichers kann somit vorzugsweise ganz oder teilweise entfallen.

Anders als bei Orthesen und Prothesen aus dem Stand der Technik ist es erfindungsgemäß folglich möglich, den wenigstens einen ersten Energiespeicher von der Bewegung des Fußteils relativ zu dem Unterschenkelteil zu entkoppeln, ohne das die zu diesem Zeitpunkt in dem ersten Energiespeicher enthaltene Energie freigesetzt wird. Die Freisetzung der Energie erfolgt erst, wenn das Freigabeelement in die Freigabestellung gebracht wird. Insbesondere kann beispielsweise in der kontrollierten Plantarflexionsphase der erste Energiespeicher mit Energie aufgeladen werden. Dann wird der Energiespeicher von der Bewegung des Fußteils relativ zu dem Unterschenkelteil entkoppelt, indem das Koppelelement in die Entkoppelstellung gebracht wird. Dabei befindet sich das Freigabeelement vorzugsweise in der Sperrstellung, so dass vorzugsweise keine oder zumindest nur wenig Energie freigegeben wird. Die Energie wird vorzugsweise während der kontrollierten Dorsalflexion und der angetriebenen Plantarflexion in dem ersten Energiespeicher gespeichert und vorzugsweise erst in der Schwungphase wieder freigegeben. Sie wirkt dann bevorzugt in Dorsalflexionsrichtung und sorgt idealerweise dafür, dass der Fuß in die optimale Position für die nächste kontrollierte Plantarflexionsphase gebracht wird, in der der erste Energiespeicher wieder mit Energie aufgeladen wird.

Das Koppelelement ist beispielsweise derart ausgebildet und angeordnet, dass es durch das Verschwenken des Unterschenkelteils relativ zu dem Fußteil um die Schwenkachse herum in die Koppelstellung oder die Entkoppelstellung bringbar ist. In dieser Ausgestaltung ist das Koppelelement beispielsweise ein Vorsprung oder ein hervorstehende Stift, der an dem Unterschenkelteil oder dem Fußteil angeordnet und bevorzugt mit diesem drehfest verbunden ist. An dem jeweils anderen Bauteil befindet sich beispielsweise ein Mitnehmer, der mit dem Vorsprung oder Stift in Kontakt bringbar ist, indem das Fußteilund das Unterschenkelteil relativ zueinander verschwenkt werden.

Ist der Vorsprung oder Stift mit dem Mitnehmer in Kontakt, befindet sich das Koppelelement in der Koppelstellung. Wird das Vorsprung oder Stift von dem Mitnehmer entfernt, indem das Fußteil relativ zudem Unterschenkelteil verschwenkt wird, befindet sich das Koppelelement dann in der Entkoppelstellung. Durch erneutes Verschwenken in die entgegengesetzte Richtung wird der Vorspung oder Stift wieder mit dem Mitnehmer in Kontakt und damit das Koppelelement wieder in die Koppelstellung gebracht, ohne dass dazu das Freigabeelement betätigt werden muss.

Vorzugsweise ist die in dem ersten Energiespeicher gespeicherte Energiemenge durch ein Verschwenken des Fußteils relativ zum Unterschenkelteil nicht beeinflussbar, wenn sich das Koppelelement in der Entkoppelstellung befindet. Es kann folglich keine Energie abgegeben werden solange sich das Freigabeelement nicht in der Freigabestellung befindet, es kann jedoch auch durch eine weitere Plantarflexion keine zusätzlich Energie in dem ersten Energiespeicher gespeichert werden, solange sich das Koppelelement in der Entkoppelstellung befindet. Alternativ dazu kann die in dem ersten Energiespeicher gespeicherte Energiemenge auch weiter vergrößerbar sein, wenn sich das Koppelelement in der Koppelstellung befindet und eine erweiterte Plantarflexion stattfindet.

Vorzugsweise weist die orthopädietechnische Gelenkeinrichtung eine elektrische Steuerung auf, die eingerichtet ist, das wenigstens eine Freigabeelement aus der Sperrstellung in die Freigabestellung zu bringen. Vorzugsweise ist auch die umgekehrte Richtung möglich, so dass mehrfach zwischen den beiden Stellungen umgeschaltet werden kann.

Die Freigabe der in dem wenigstens einen Energiespeicher gespeicherten Energie kann somit insbesondere automatisiert und beispielsweise in Abhängigkeit zeitlicher oder anderer Parameter ablaufen.

Darunter, dass die elektrische Steuerung eingerichtet ist, das wenigstens eine Freigabeelement aus der Sperrstellung in die Freigabestellung zu bringen und umgekehrt, ist hierbei insbesondere zu verstehen, dass die elektrische Steuerung beispielsweise zumindest einen Aktuator ansteuert, der dann das Freigabeelement insbesondere mechanisch aus der Sperrstellung in die Freigabestellung bringt und umgekehrt. Dazu weist die elektrische Steuerung wenigstens eine elektronische Datenverarbeitungseinrichtung, bevorzugt einen Mikroprozessor, und wenigstens einen Datenspeicher auf. Alternativ kann auch lediglich ein Zugriff auf einen derartigen Datenspeicher bereitgestellt werden.

Vorzugsweise weist die orthopädietechnische Gelenkeinrichtung wenigstens einen Sensor auf, der insbesondere ein Druck-, Lage-, Kraft- , Weg-, Winkel-, Relativwinkel- und/oder Beschleunigungssensor und/oder ein Sensor ist, mit dem eine Energiemenge detektierbar ist, die in dem Energiespeicher enthalten ist. Dieser Sensor ist vorzugsweise eingerichtet wenigstens einen Messwert zu erfassen und an die elektrische Steuerung zu übermitteln.

Auf diese Weise ist es insbesondere möglich, mittels des zumindest einen Sensors Informationen dahingehend zu erhalten, in welcher Phase eines Gangzyklus sich der Träger der orthopädietechnischen Gelenkeinrichtung befindet. Hierzu erfasst der zumindest eine Sensor vorzugsweise kontinuierlich Messwerte und übermittelt diese an die elektrische Steuerung. Alternativ dazu erfasst der zumindest eine Sensor lediglich in bestimmten Zeitintervallen Messwerte und übermittelt sie an die elektrische Steuerung. Der Sensor beinhaltet vorzugsweise eine eigene Datenverarbeitungseinrichtung, die es ihm beispielsweise ermöglicht, die erfassten Messwerte, deren Ableitungen oder Integrale zu verarbeiten, beispielsweise mit hinterlegten Grenzwerten zu vergleichen, und die Messwerte, deren Ableitungen oder Integrale nur dann an die elektrische Steuerung zu übermitteln, wenn der hinterlegte Grenzwert über- oder unterschritten wird.

So kann beispielsweise mittels wenigstens eines Drucksensors, der beispielsweise an einer im Betrieb der Orthese dem Boden zugewandten Unterseite des Fußteils, bevorzugt im Fersenbereich, angeordnet ist, detektiert werden, ob die Fußheberorthese Bodenkontakt hat. So ist insbesondere feststellbar, ob sich die Fußheberorthese in der Schwungphase des Gangzyklus befindet.

Vorzugsweise weist die orthopädietechnische Gelenkeinrichtung zumindest zwei Sensoren, insbesondere Drucksensoren, auf. Diese sind vorzugsweise in einem Fersenbereich des Fußteils, welcher im angelegten Zustand im Bereich der Ferse eines Trägers liegt, und einem Vorfußbereich des Fußteils, welcher im angelegten Zustand im Bereich des Vorfußes des Trägers anliegt, angeordnet. Dies hat den Vorteil, dass nicht nur die völlige Kontaktlosigkeit zum Boden detektiert werden kann, sondern auch unterschiedliche Phasen unterscheiden werden können, indem beispielsweise ermittelt wird, wie sich die Belastung vom Fersenbereich zum Vorfußbereich verschiebt. Es kann alternativ oder zusätzlich dazu detektiert werden, wenn Bodenkontakt nur noch im Vorfußbereich vorliegt, wie beispielsweise vor dem "toe-off" im Gangzyklus. Durch die vorteilhafte Verwendung von zumindest zwei Drucksensoren ist somit ein deutlich detaillierteres Abbilden des Gangzyklus möglich.

Vorzugsweise verfügt die orthopädietechnische Gelenkeinrichtung über wenigstens zwei unterschiedliche Sensoren.

Mittels zumindest eines besonders bevorzugten Sensors lassen sich der Winkel und/oder die Winkeländerung beim Verschwenken des Fußteils um die Schwenkachse, relativ zu dem Unterschenkelteil bestimmen. So kann der Gangzyklus besonders einfach und detailliert abgebildet werden.

Ein solcher Sensor ließe sich beispielsweise durch zumindest zwei Lagesensoren bilden, wobei zumindest ein Lagesensor am Fußteil und zumindest ein Lagesensor am Unterschenkelteil angeordnet ist. Durch den Abstand der beiden Sensoren zueinander ist, bei bekannter Position der Lagesensoren an Fußteil und Unterschenkelteil und bekannter Schwenkachse, der Winkel zwischen dem Fußteil und dem Unterschenkelteil bestimmbar.

Die Übermittlung des Messwertes kann insbesondere über ein Datenkabel oder über drahtlose Übertragungsmittel, wie beispielsweise per Funk, WLAN oder Bluetooth erfolgen.

Vorzugsweise ist die elektrische Steuerung eingerichtet, das wenigstens eine Freigabeelement, in Abhängigkeit von dem wenigstens einen übermittelten Messwert, aus der Sperrstellung in die Freigabestellung und/oder umgekehrt zu bringen. So wird verhindert, dass zu nicht gewünschten Zeitpunkten Energie freigegeben wird.

Um den Träger der orthopädietechnischen Gelenkeinrichtung bei der in der Schwungphase stattfindenden Dorsalflexion zu unterstützen, ist es insbesondere vorteilhaft, die Freigabe der Energie aus dem wenigstens einen Energiespeicher auf den Beginn der Schwungphase, also insbesondere den Zeitpunkt des Abhebens des Fußes vom Boden ("toe-off") abzustimmen.

Weiterhin kann beispielsweise im Zeitpunkt der maximalen Plantarflexion in der kontrollierten Plantarflexionsphase das Freigabeelement von der Freigabestellung in die Sperrstellung gebracht werden, sodass die während der kontrollierten Plantarflexion in dem wenigstens einen ersten Energiespeicher gespeicherte Energie nicht während der Dorsalflexionsphase ganz oder teilweise freigesetzt wird.

Es ist daher vorteilhaft, wenn die elektrische Steuerung eingerichtet ist, anhand des zumindest einen übermittelten Messwertes einen Verlust des Bodenkontakts der orthopädietechnischen Gelenkeinrichtung zu detektieren und das Freigabeelement aus der Sperrstellung in die Freigabestellung zu bringen, wenn der Verlust des Bodenkontakts detektiert wurde.

Weiterhin ist es vorteilhaft, wenn die elektrische Steuerung eingerichtet ist, das Koppelelement erst dann von der Koppelstellung in die Entkoppelstellung zu bringen, wenn sich das Freigabeelement in der Sperrstellung befindet.

Die Erhöhung der Energiemenge in dem wenigstens einen Energiespeicher erfolgt insbesondere in der kontrollierten Plantarflexionsphase. Mit Erreichen der maximalen Plantarflexion ist folglich der maximale in dieser Phase mögliche Energieeintrag erfolgt. Für den Fall, dass es sich bei dem wenigstens einen Energiespeicher um ein Federelement handelt, ist es mit Erreichen der maximalen Plantarflexion vorteilhaft, wenn der Energiespeicher mittels des Koppelelementes von der Bewegung des Fußteils relativ zum Unterschenkelteil entkoppelt wird, da die sich anschließende kontrollierte Dorsalflexion wieder zu einer Entladung des wenigstens einen Energiespeichers führen würde. Die Entkopplung des Federelements während sich das Freigabeelement in der Freigabestellung befindet, würde allerdings insbesondere zu einer teilweisen oder vollständigen Freisetzung der Energie durch Entspannung des Federelementes führen. Aus diesem Grund ist es vorteilhaft, zunächst das Freigabeelement von der Freigabestellung in die Sperrstellung zu bringen und erst wenn dies erfolgt ist, das Koppelelement von der Koppelstellung in die Entkoppelstellung zu bringen. Das Freigabeelement wird vorzugsweise in die Sperrstellung gebracht, sobald beispielsweise in der Schwungphase die gesamte in dem Energiespeicher gespeicherte Energie freigegeben wurde.

Vorteilhafterweise weist die orthopädietechnische Gelenkeinrichtung wenigstens einen zweiten Energiespeicher auf, der derart eingerichtet ist, dass ein Verschwenken des Fußteils relativ zum Unterschenkelteil um die Schwenkachse in einer Dorsalflexionsrichtung zu einer Erhöhung der in dem zweiten Energiespeicher gespeicherten Energiemenge führt.

Ein solcher zweiter Energiespeicher ist insbesondere hinsichtlich einer Unterstützung des Trägers der Orthese während der angetriebenen Plantarflexionsphase von Vorteil. So kann insbesondere während der kontrollierten Dorsalflexionsphase Energie in den wenigstens einen zweiten Energiespeicher aufgenommen werden, welche insbesondere in der sich anschließenden angetriebenen Plantarflexionsphase wieder freigesetzt wird.

Weiterhin ist es durch diese Kombination insbesondere möglich, die Energie aus dem zumindest einen ersten und dem zumindest einen zweiten Energiespeicher synergistisch zu nutzen, da das aus dem Stand der Technik bekannte "gegeneinander Arbeiten" entfällt. So wird vorzugsweise zunächst die Energie aus dem zumindest einen zweiten Energiespeicher in der angetriebenen Plantarflexionsphase freigesetzt werden und anschließend, nach dem "toe-off", die Energie aus dem zumindest einen ersten Energiespeicher freigegeben werden, indem das Freigabeelement in die Freigabestellung gebracht wird.

Vorzugsweise handelt es sich bei dem wenigstens einen ersten Energiespeicher und/oder dem wenigstens einen zweiten Energiespeicher um ein Federelement und/oder eine hydraulische und/oder eine pneumatische Vorrichtung.

Bei dem Federelement handelt es sich insbesondere um eine Schraubenfeder oder eine Torsionsfeder. Weiterhin ist es möglich, dass mittels einer hydraulischen Vorrichtung Energie in einem solchen Federelement gespeichert wird. Darüber hinaus ist es ebenfalls möglich, dass mittels einer Pumpe oder eines Kompressors Energie durch Kompression eines Gases gespeichert wird. Es ist darüber hinaus insbesondere möglich, dass in einer orthopädietechnischen Gelenkeinrichtung mehrere unterschiedliche Arten von ersten und/oder zweiten Energiespeichern vorgesehen sind.

Bei mechanischen Energiespeichern, insbesondere Federelementen, ist das Freigabeelement beispielsweise ein Ratschenelement oder Freilaufelement. Wird ein pneumatischer oder hydraulischer Energiespeicher verwendet, ist das Freigabeelement vorzugsweise ein Ventil, das insbesondere elektrisch oder elektronisch geschaltet werden kann. Das Koppelelement kann auch ein Fluid sein.

In einer Variante der Erfindung ist Koppelelement als Fluid-Ventil-Kombination ausgebildet ist. Damit ist es möglich, eine Dorsalextension zu einem beliebigen, frei wählbaren Zeitpunkt auszuführen.

Wenn der Gelenkeinrichtung zwei Rotationshydrauliken zugeordnet sind, bevorzugt medial und lateral, kann eine wechselseitige Energiespeicherung und Energieabgabe in unterschiedlichen Bewegungsrichtungen, nämlich Plantarflexion und Dorsalextension, durchgeführt werden. Dadurch lässt sich eine erweiterte Dorsalextension durchführen, ohne das die Beeinflussung der Plantarflexion davon beeinträchtigt wird.

Mit Hilfe der beiliegenden Zeichnungen werden nachfolgend einige Ausführungsbeispiele der vorliegenden Erfindung erläutert. Es zeigen
- Figuren 1, 3, 5, 7 und 9 -: schematische Darstellungen unterschiedlicher Ausführungen von orthopädietechnischen Gelenkeinrichtungen,
- Figuren 2, 4, 6, 8 und 10 -: die in den jeweils vorangegangenen Figuren dargestellten Ausführungsbeispiele in unterschiedlichen Bewegungsphasen,
- Figuren 11 und 12 -: schematische Darstellungen der hydraulischen/pneumatischen Systeme
- Figur 13 -: die schematische Darstellung einer orthopädietechnischen Gelenkeinrichtung,
- Figur 14 -: die schematische Darstellung des Energieverlaufs in den Energiespeicherelementen gemäß dem Ausführungsbeispiel aus Figur 2 und
- Figuren 15 bis 20 -: verschieden Phasen eines Schrittes bei einem weiteren Ausführungsbeispiel der vorliegenden Erfindung.

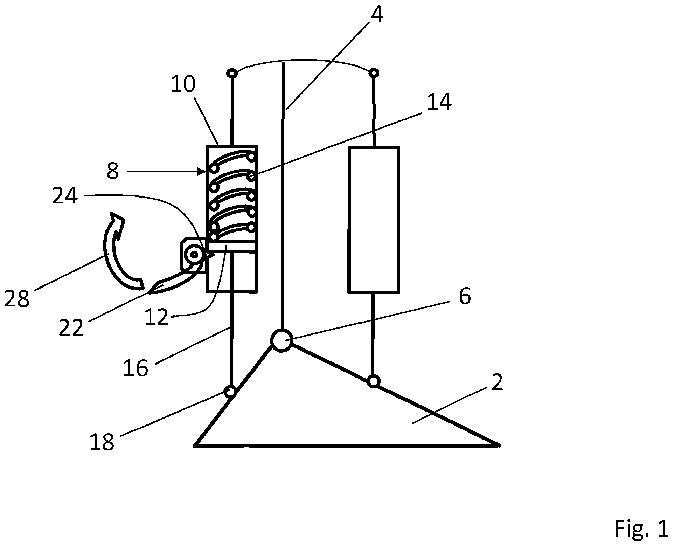

Figur 1 zeigt die schematische Darstellung einer orthopädietechnischen Einrichtung gemäß einem ersten Ausführungsbeispiel der vorliegenden Erfindung. Sie verfügt über ein Fußteil 2, das an einem Unterschenkelteil 4 um eine Schwenkachse 6 herum schwenkbar gelagert ist. Sie verfügt zudem über einen ersten Energiespeicher 8, der im gezeigten Ausführungsbeispiel einen in einem Zylinder 10 beweglich gelagerten Kolben 12 aufweist, der durch eine Feder 14 vorgespannt ist. Am Kolben 12 befindet sich ein Stößel 16, der über ein Koppelelement 18 mit dem Fußteil 2 verbunden ist.

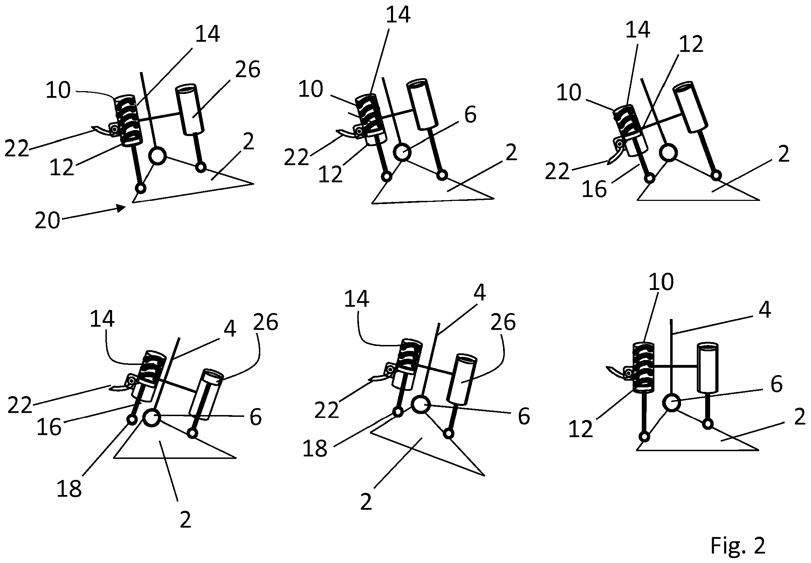

In Figur 2 ist diese Ausführungsform einer orthopädietechnischen Gelenkeinrichtung in unterschiedlichen Bewegungsphasen eines Schrittes dargestellt. Links oben ist die Situation beim Fersenauftritt dargestellt. Das Fußteil 2 ist gegen die horizontale Verkippung gesperrt und eine Ferse 20 bekommt Bodenkontakt beim Aufsetzen des Fußes. Die Feder 14 ist soweit wie möglich entspannt und der Kolben 12 befindet sich am unteren Ende des Zylinders 10. Rechts daneben ist die Situation während des Abrollens des Fußes, also der kontrollierten Plantarflexion gezeigt. Das Fußteil 2 ist gegenüber der links dargestellten Situation in Plantarflexionsrichtung um die Schwenkachse 6 herum verschwenkt worden. Der Kolben 12 wurde im Zylinder 10 nach oben bewegt und hat dadurch die Feder 14 zusammengedrückt und somit den Energiegehalt in diesem Energiespeicher erhöht. In der rechts oben dargestellten Situation liegt das Fußteil vollflächig auf dem Boden auf. Es handelt sich um die in diesem Schritt maximal plantarflektierte Stellung. Der Stößel 16 hat den Kolben 12 im Zylinder 10 weitest möglich nach oben verschoben, sodass die Feder 14 stark komprimiert wurde. In dieser Situation wird ein Freigabeelement 22, das sich in den oben links und Mitte links in Figur 2 dargestellten Situationen in einer Freigabestellung befand, aus dieser Freigabestellung in die Sperrstellung gebracht. Diese ist in Figur 1 detaillierter dargestellt. Das Freigabeelement verfügt über eine Sperrklinke 24, die den Kolben 12 im Zylinder 10 in der gezeigten Position befestigt. Bei der anschließenden kontrollierten Dorsalflexion, die in Figur 2 unten links dargestellt ist, wird das Fußteil 2 gegenüber dem Unterschenkelteil 4 um die Schwenkachse 6 herum verschwenkt, wobei es jedoch nicht zu einer Entspannung der Feder 14 kommt. Die im Energiespeicher 14 gespeicherte Energie bleibt erhalten, da das Freigabeelement 22 sich in der Sperrstellung befindet. Man erkennt zudem, dass das Koppelelement 18 den Stößel 16 vom Fußteil 2 entkoppelt hat.

Durch einen zweiten Energiespeicher 26 kann im gezeigten Ausführungsbeispiel Energie für eine aktive Plantarflexionsbewegung bereitgestellt werden, die in Figur 2 unten Mitte dargestellt ist. Das Fußteil 2 wird gegen das Unterschenkelteil 4 um die Schwenkachse 6 herum verschwenkt und das Koppelelement 18 kommt wieder in Kontakt mit dem Fußteil 2. In diesem Fall kann das Freigabeelement 22 dem in Figur 1 gezeigten Pfeil 28 folgend aus der Sperrstellung in die Freigabestellung gebracht werden, sodass Energie aus der Feder 14 abgeführt werden kann, wodurch der Kolben 12 innerhalb des Zylinders 10 nach unten bewegt wird. Diese Situation ist in Figur 2 unten rechts dargestellt. Das Fußteil 2 ist gegen das Unterschenkelteil 4 um die Schwenkachse 6 herum verschwenkt und der Vorfußbereich angehoben.

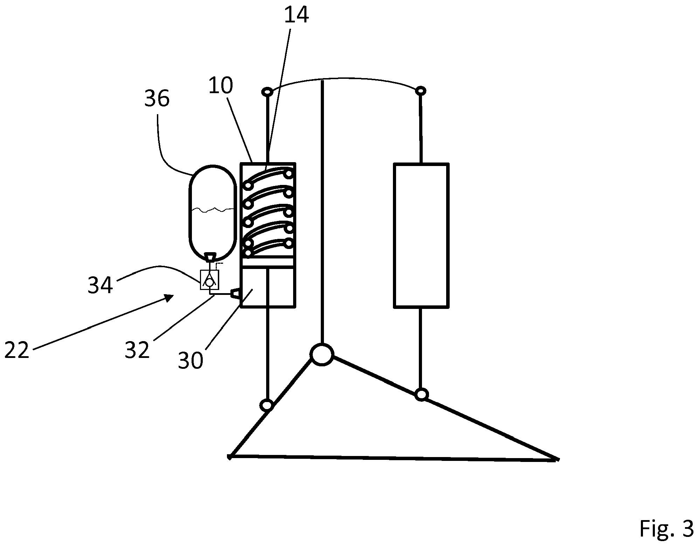

Figur 3 zeigt eine Darstellung ähnlich der Figur 1. Konstruktiver Unterschied ist die Ausgestaltung des Freigabeelementes 22. In dem Zylinder 10 befindet sich unterhalb der Feder 14 ein Volumen 30, das über eine Fluidleitung 32, in der sich ein schaltbares Ventil 34 befindet, mit einem Ausgleichsreservoir 36 verbunden ist.

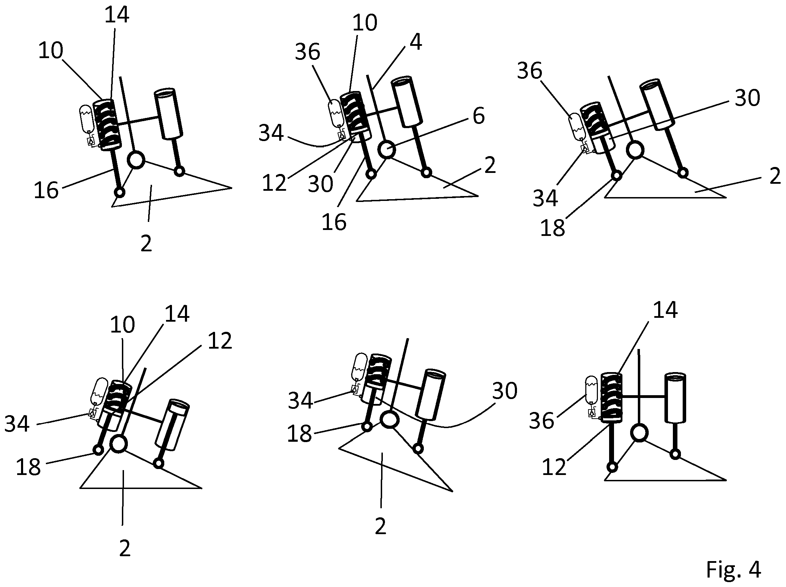

Figur 4 zeigt die Ausgestaltung aus Figur 3 in den Positionen gemäß Figur 2. Beim Fersenauftritt (oben links) des Fußteils 2 ist der Stößel 16 soweit es geht aus dem Zylinder 10 heraus verschoben, sodass die Feder entspannt ist. Beim anschließenden Überrollen und der kontrollierten Plantarflexion wird das Fußteil 2 relativ zum Unterschenkelteil 4 um die Schwenkachse 6 herum verschwenkt und, wie in der Darstellung oben Mitte dargestellt, der Stößel 16 mit dem daran angeordneten Kolben 12 innerhalb des Zylinders 10 nach oben verschoben. Dabei ist das schaltbare Ventil 34 geöffnet, sodass Fluid aus dem Ausgleichsreservoir 36 in das Volumen 30 strömen kann. Dies geschieht bis zur maximal plantarflektierten Stellung, die in Figur 4 oben rechts dargestellt ist. In diesem Zustand wird das schaltbare Ventil 34 geschlossen, sodass kein Fluid mehr aus dem Volumen 30 in das Ausgleichsreservoir 36 strömen kann. Im gezeigten Ausführungsbeispiel wird gleichzeitig das Koppelelement 18 vom Fußteil 2 entkoppelt. Figur 4 zeigt in der Darstellung unten links die maximal dorsalflektierte Stellung. Das Koppelelement 18 ist nicht in Eingriff mit dem Fußteil 2 und, da das schaltbare Ventil 34 noch immer geschlossen ist, ist auch der Kolben 12 im Zylinder 10 nicht nach unten verschoben worden, sodass der Energiespeicher, der als Feder 14 ausgebildet ist, weiterhin die Energie speichert. Dies geschieht zur mittleren Darstellung der unteren Reihe in Figur 4. Das Kopplungselement 18 gerät wieder in Eingriff mit dem Fußteil 2 und das schaltbare Ventil 34 wird geöffnet. Dadurch kann die Feder 14 entspannt werden, sodass der Kolben 12 nach unten verschoben und ein Fluid aus dem Volumen 30 in das Ausgleichsreservoir 36 geleitet werden kann.

Das schaltbare Ventil 34 kann auch als Drosselventil verwendet werden, sodass die Abgabe der Energie aus dem Energiespeicher, also der Feder 14, gedämpft erfolgen kann. Dies ist selbstverständlich auch in allen anderen Ausgestaltungen möglich, um ein abruptes Abgeben der Energie zu vermeiden.

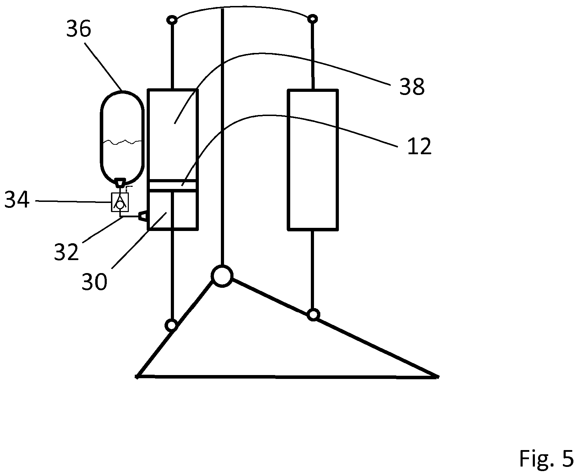

Figur 5 zeigt eine Darstellung ähnlich den Figuren 1 und 3. Konstruktiver Unterschied zur Darstellung aus Figur 3 ist einzig die Form des Energiespeichers. Statt der Feder 14 ist ein Druckspeicher 38 vorhanden, in dem ein kompressibles Medium enthalten ist. Das sich unter dem Kolben 12 befindende Volumen 30 ist wieder über die Fluidleitung 32 und das schaltbare Ventil 34 mit dem Ausgleichsreservoir 36 verbunden.

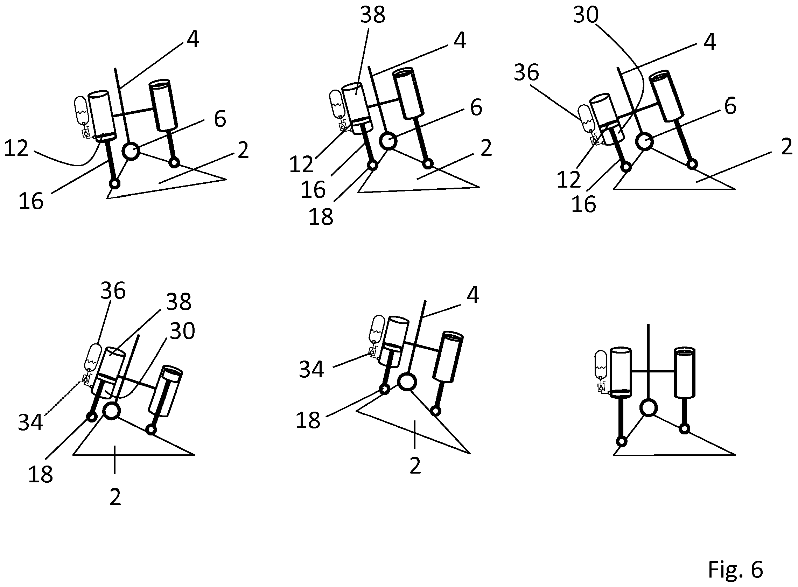

Figur 6 zeigt die in Figur 5 dargestellte Ausführungsform in den unterschiedlichen Bewegungsphasen. Nach dem Fersenauftritt (oben links) wird das Fußteil 2 relativ zum Unterschenkelteil 4 um die Schwenkachse 6 herum in einer Plantarflexionsrichtung verschwenkt. Dabei wird der Stößel 16 und damit auch der Kolben 12 nach oben verschoben. Das Volumen 30 wird durch Fluid aus dem Ausgleichsreservoir 36 gefüllt. Der Druckspeicher 38, in dem sich das kompressible Medium befindet, wird komprimiert, sodass Energie in Form von Druck gespeichert wird.

Anschließend wird sowohl das Kopplungselement 18 außer Eingriff mit dem Fußteil 2 gebracht als auch das schaltbare Ventil 34 geschlossen, sodass kein Fluid mehr aus dem Volumen 30 in das Ausgleichsreservoir 36 fließen kann. Bei der in Figur 6 unten links dargestellten Dorsalflexion kann folglich der Druck aus dem Druckspeicher 38 nicht abgebaut werden. Erst wenn in der aktiv gesteuerten Plantarflexion (Figur 6 Mitte unten) der Fußteil 2 wieder die gezeigte Position relativ zum Unterschenkelteil 4 aufweist, wird das Koppelelement 18 wieder in Eingriff gebracht und das schaltbare Ventil 34 geöffnet.

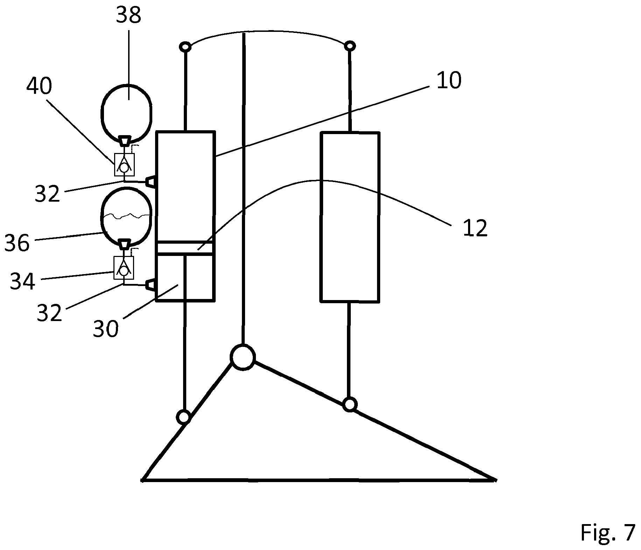

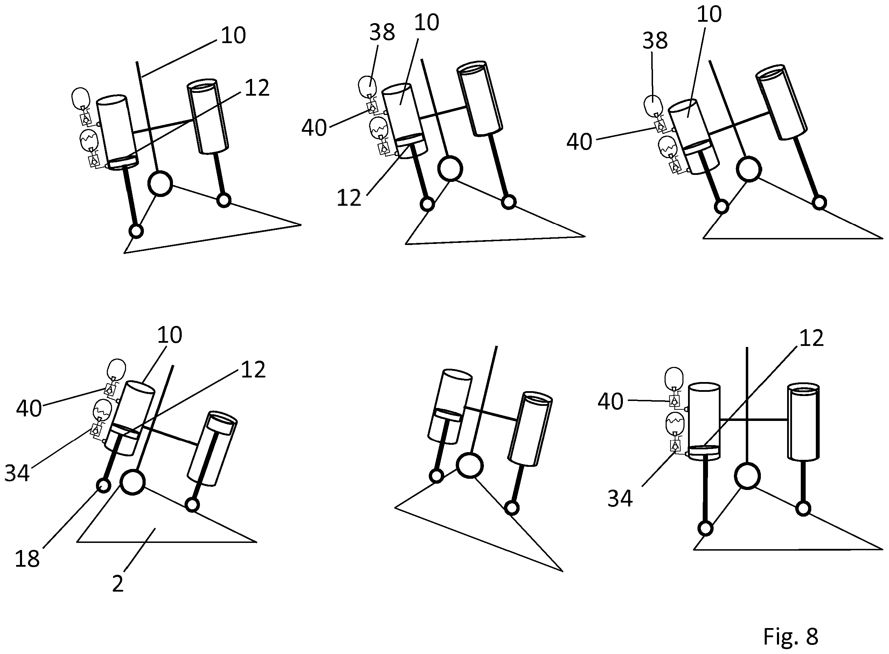

Figur 7 zeigt eine nochmals geänderte Ausführungsform. Das Volumen 30 unterhalb des Kolbens 12 ist über die Fluidleitung 32 und das schaltbare Ventil 34 mit dem Ausgleichsreservoir 36 verbunden. Der Druckspeicher 38 ist nun außerhalb des Kolbens 10 angeordnet und ebenfalls über eine Fluidleitung 32 und ein zweites schaltbares Ventil 40 mit dem Kolben 10 verbunden. Figur 8 zeigt diese Ausführungsformen in unterschiedlichen Bewegungsphasen. Die Funktionsweise entspricht der zur Figur 6 erklärten Funktionsweise. Durch das Verschieben des Kolbens 12 innerhalb des Zylinders 10 nach oben wird bei geöffnetem zweiten schaltbaren Ventil 40 ein Fluid aus dem Zylinder 10 in den Druckspeicher 38 gedrückt.

In der unteren Zeile der Figur 8 ist dargestellt, wie das zweite schaltbare Ventil 40 ebenso wie das schaltbare Ventil 34 geschlossen ist, sodass der Kolben 12 innerhalb des Zylinders 10 nicht bewegt werden kann. Das Koppelelement 18 ist außer Eingriff mit dem Fußteil 2.

Erst wenn das schaltbare Ventil 34 und das zweite schaltbare Ventil 40 geöffnet werden, wie dies in Figur 8 Mitte unten und unten rechts dargestellt ist, kann der Kolben 12 wieder nach unten verschoben werden.

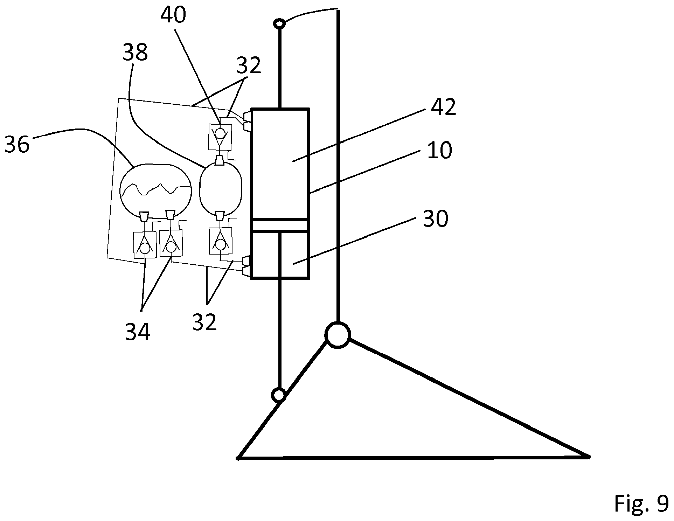

Figur 9 zeigt eine weitere Ausgestaltung einer orthopädietechnischen Gelenkeinrichtung. Der Zylinder 10 verfügt über zwei Kammern, die durch das Volumen 30 und das Volumen 42 gebildet werden. Die Gelenkeinrichtung verfügt über den Druckspeicher 38 sowie das Ausgleichsreservoir 36. Beide sind über jeweils eine Fluidleitung 32 sowohl mit dem Volumen 30 als auch mit dem Volumen 42 verbunden. In jeder dieser Fluidleitungen 32 befindet sich ein schaltbares Ventil 34.

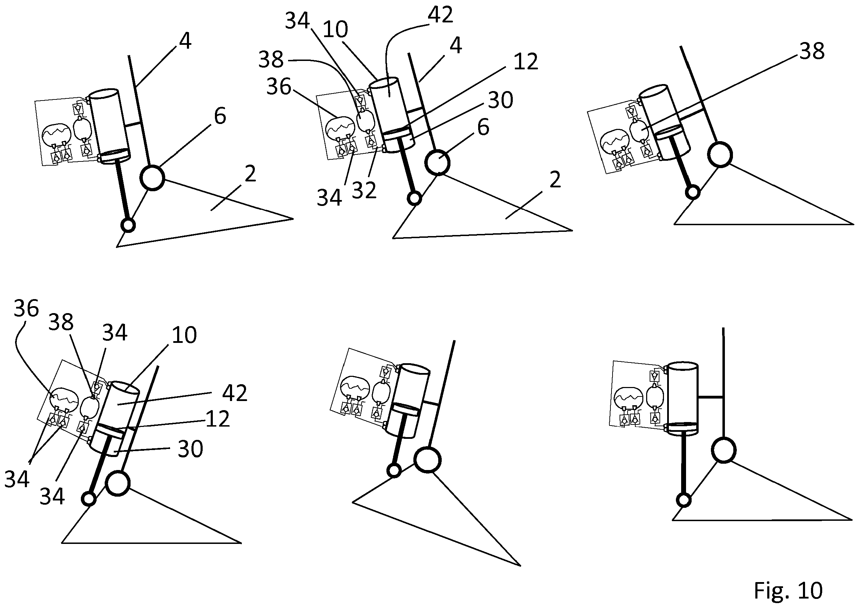

Figur 10 zeigt die Ausgestaltung aus Figur 9 in den unterschiedlichen Bewegungsphasen. Nach dem Fersenauftritt (oben links) wird das Fußteil 2 relativ zum Unterschenkelteil 4 um die Schwenkachse 6 herum verschwenkt. Dadurch wird der Kolben 12 in dem Zylinder 10 nach oben verschoben. Das schaltbare Ventil 34, das das Volumen 42 mit dem Druckspeicher 38 verbindet, ist ebenso geöffnet, wie das schaltbare Ventil 34, durch das die Fluidleitung 32 geöffnet wird, die das Volumen 30 mit dem Ausgleichsreservoir 36 verbindet. Bei dieser Bewegung wird der Druck innerhalb des Druckspeichers 38 folglich erhöht. Bei der anschließenden Dorsalflexion (unten links) sind die beiden Ventile 34, die die Volumina 30, 42 mit dem Druckspeicher 38 verbinden, geschlossen, während die anderen beide Ventile 34, durch die die Volumina 30, 42 mit dem Ausgleichsreservoir 36 verbunden sind, geöffnet sind. Auf diese Weise kann der Kolben 12 innerhalb des Zylinders 10 verschoben werden, ohne dass sich der Druck innerhalb des Druckspeichers 38 verändert. Die Ventile 34 sind folglich Teile des Koppelelementes und/oder der Freigabeeinrichtung.

Die gezeigten Ausführungsformen bis auf die der Figuren 9, 10 und 12 verfügen zudem über den zweiten Energiespeicher 26. Er kann auf unterschiedliche Weisen ausgebildet sein und ist vorzugsweise so angeordnet, dass bei einer Dorsalflexionsbewegung die Menge der gespeicherten Energie zunimmt. Sie kann bei einer gesteuerten und aktivierten Plantarflexionsbewegung abgegeben werden. Vorzugsweise ist auch dieser Energiespeicher ein hydraulisches oder ein hydraulisch-pneumatisches System. Selbstverständlich sind auch andere Energiespeicherausführungen möglich.

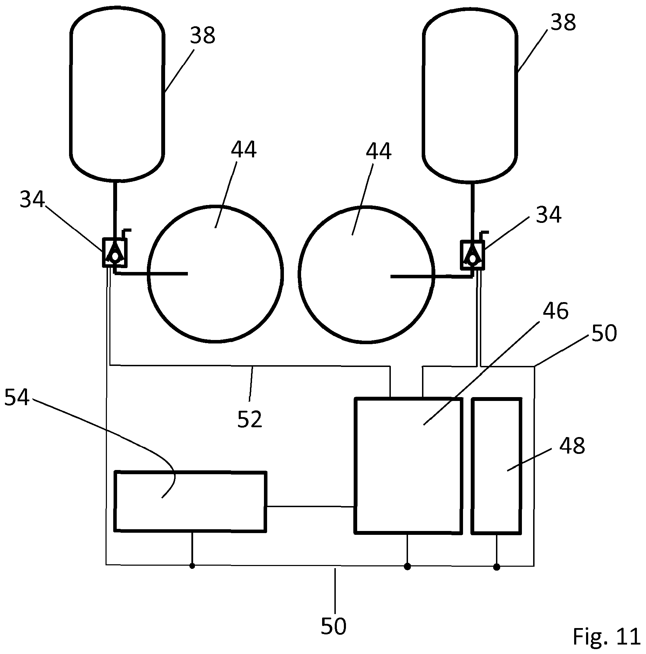

Figur 11 zeigt eine schematische Darstellung einer entsprechenden orthopädietechnischen Gelenkeinrichtung. Sie verfügt über zwei Druckspeicher 38, von denen der in Figur 11 linke Druckspeicher 38 dem ersten Energiespeicher 8 entspricht. Jeweils eine Rotationshydraulik 44 ist jedem der beiden Druckspeicher 38 zugeordnet, um aus der Bewegung des Fußteils relativ zum Unterschenkelteil Energie speichern zu können. Die Vorrichtung verfügt über eine elektronische Datenverarbeitungseinrichtung 46, die als Mikroprozessor ausgebildet ist. Sie wird über eine Spannungsversorgung 48 mit elektrischer Energie versorgt. Über elektrische Leitungen 50 wird elektrische Energie auch zu den schaltbaren Ventilen 34 geleitet.

Diese sind über Steuerleitungen 52 zudem mit der elektronischen Datenverarbeitungseinrichtung 46 verbunden, durch die sie Steuersignale erhalten. Die Vorrichtung verfügt zudem über eine Sensorik 54, die mehrere gegebenenfalls unterschiedliche Sensoren aufweisen kann, deren Messwerte zur Steuerung der Vorrichtung verwendet werden.

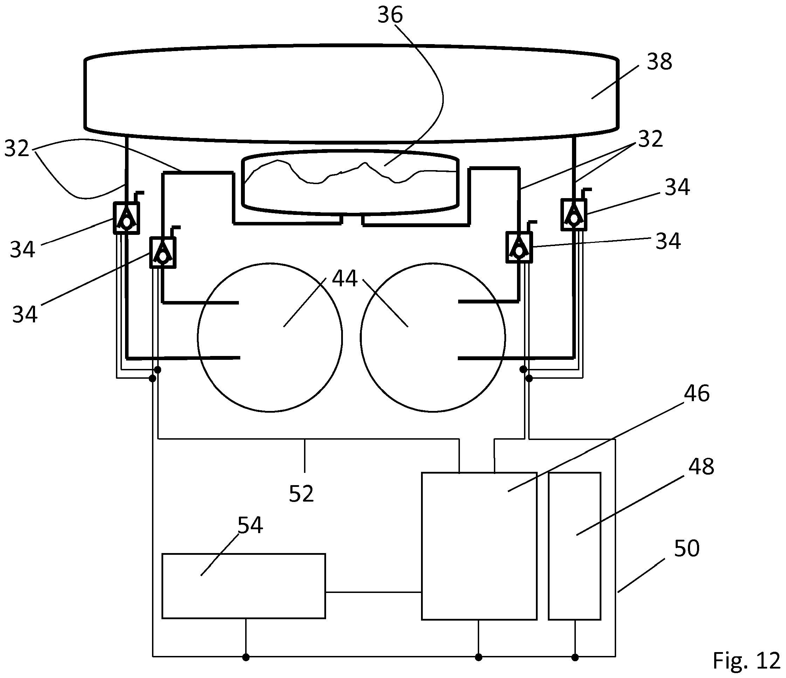

Figur 12 zeigt eine andere Ausgestaltung. Sie verfügt nur über einen einzigen Druckspeicher 38 sowie über ein Ausgleichsreservoir 36. Die beiden Rotationshydrauliken 44 sind über jeweils eine Fluidleitung 32 sowohl mit dem Druckspeicher 38 als auch mit dem Ausgleichsreservoir 36 verbunden. Jeweils ein schaltbares Ventil 34 ist in jeder dieser Leitungen vorhanden.

Die schaltbaren Ventile sind über Steuerleitungen 52 mit der elektronischen Datenverarbeitungseinrichtung 46 und über elektrische Leitungen 50 mit der Spannungsversorgung 48 verbunden. Auch diese Vorrichtung verfügt über die entsprechende Sensorik.

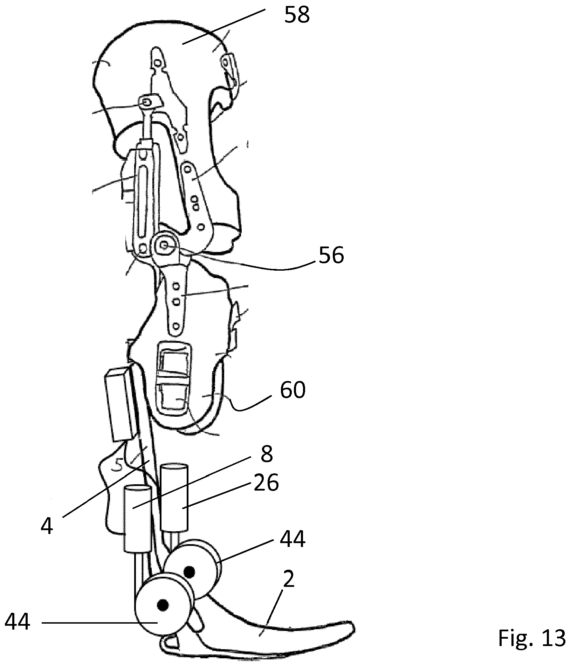

Figur 13 zeigt die schematische Ansicht einer entsprechenden orthopädietechnischen Gelenkeinrichtung, die als Knie-, Knöchel-, Fuß-Orthese ausgebildet ist. Sie verfügt über ein Fußteil 2, den Unterschenkelteil 4 sowie einen ersten Energiespeicher 8 und einen zweiten Energiespeicher 26. Im Bereich des Knies befindet sich ein Gelenk 56. Darüber befindet sich eine Oberschenkelschale 58 sowie eine Unterschenkelschale 60 zum Anlegen an den Oberschenkel oder den Unterschenkel. Im Knöchelbereich sind zwei Rotationshydrauliken 44 vorgesehen, eine medial und die andere lateral, die mit den Energiespeichern 8, 26 verbunden sind.

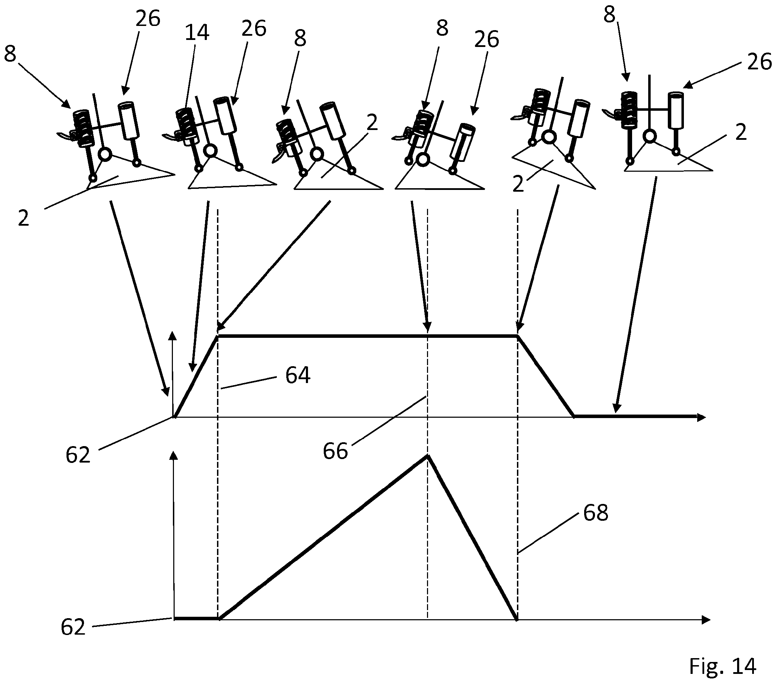

Figur 14 zeigt schematisch den Verlauf der gespeicherten Energiemenge im ersten Energiespeicher 8 und im zweiten Energiespeicher 26. Im oberen Bereich der Figur 14 sind die Darstellungen aus Figur 2 verkleinert dargestellt. Man erkennt jeweils das Fußteil 2 mit dem ersten Energiespeicher 8 und dem zweiten Energiespeicher 26.

Im unteren Bereich zeigen die zwei Diagramme schematisch die Menge der jeweils gespeicherten Energie. Dabei bezeichnet die obere Linie die im ersten Energiespeicher 8 gespeicherte Energie, während die untere Linie die im zweiten Energiespeicher 26 gespeicherte Energie darstellt.

Beim Fersenauftritt 62, der in der ersten Zeile der Figur 14 durch die erste Darstellung gezeigt wird, ist weder im ersten Energiespeicher 8 noch im zweiten Energiespeicher 26 Energie enthalten. Dies ändert sich bei der folgenden kontrollierten Plantarflexion, während der im ersten Energiespeicher 8 Energie gespeichert wird. Sie verläuft bis zur Maximum-Plantarflexion 64, die durch die erste gestrichelte Linie in Figur 14 dargestellt ist. Man erkennt in der zweiten Darstellung von links in der ersten Zeile von Figur 14, dass das Fußteil 2 abgesenkt wird und die Feder 14 im ersten Energiespeicher 8 zusammengedrückt wird. Die dritte Darstellung in Figur 14 oben zeigt die maximal plantarflektierte Stellung. Wie bereits bezüglich in Figur 2 beschrieben, wird in diesem Moment das Freigabeelement 22 aus der Freigabestellung in die Sperrstellung gebracht. Zudem wird, wie in Figur 2 dargestellt, das Koppelelement 18 vom Fußteil 2 entkoppelt.

Da das Koppelelement 18 entkoppelt ist und das Freigabeelement 22 sich in der Sperrstellung befindet, wird bei den folgenden Bewegungen, also der kontrollierten Dorsalflexion und der aktiven Plantarflexion, während der das Freigabeelement 22 in der Sperrstellung bleibt, das Energieniveau im ersten Energiespeicher 8 nicht verändert.

In dem untersten Diagramm der Figur 14 wird die Energiemenge im zweiten Energiespeicher 26 dargestellt. Vom Fersenauftritt 62 bis zur Maximum-Plantarflexion wird keine Energie gespeichert. Während der kontrollierten Dorsalflexion bis zur maximal dorsalflektierten Stellung 66 wird im zweiten Energiespeicher 26 Energie gespeichert, die bei der anschließenden aktiven Plantarflexion wieder abgegeben wird und dem Abstoßen den Fußes dient. Dies geschieht bis zum Abheben 68 des Fußes, das in der obersten Zeile der Figur 14 durch die zweite Darstellung von rechts dargestellt ist.

Danach befindet sich der Fuß in der Schwungphase, in der im gezeigten Ausführungsbeispiel das Koppelelement 18 wieder den Stößel 16 am Fußteil 2 angekoppelt hat und das Freigabeelement 22 wieder in die Freigabestellung gebracht wird. Dadurch kann die in dem ersten Energiespeicher 9 gespeicherte Energie abgegeben werden, wodurch das Fußteil 2 gegen das Unterschenkelteil 4 um die Schwenkachse 6 herum verschwenkt wird. Die endgültige Position, in der der Vorfußbereich angehoben ist, ist in der rechten Darstellung in der oberen Zeile von Figur 14 gezeigt. In diesem Zustand befindet sich weder in dem ersten Energiespeicher 8 noch im zweiten Energiespeicher 26 Energie und der Schritt beginnt von vorn.

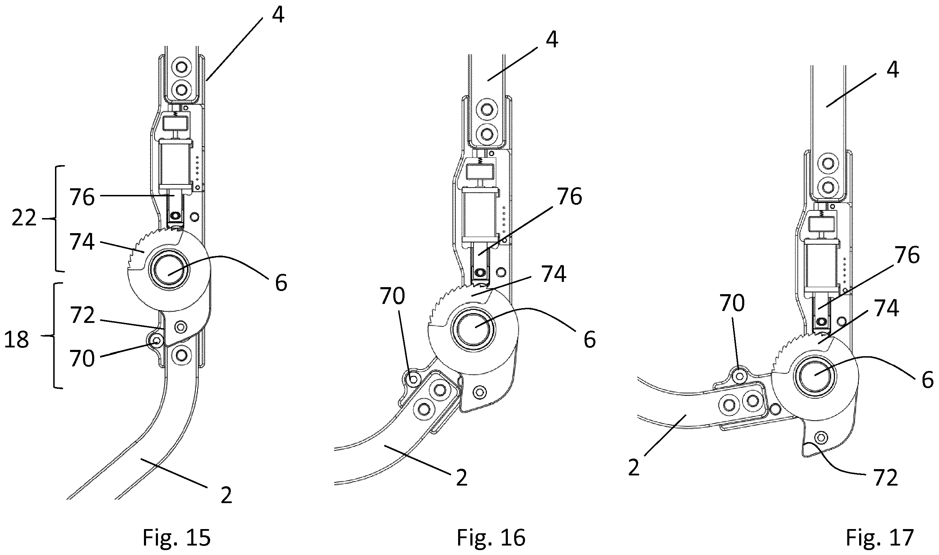

Die Figuren 15 bis 20 zeigen eine orthopädietechnische Gelenkeinrichtung gemäß einem weiteren Ausführungsbeispiel der vorliegenden Erfindung. An dem Fußteil 2 ist der Unterschenkelteil 4 um eine Schwenkachse 6 schwenkbar angeordnet. Der erste Energiespeicher ist eine nicht dargestellte Feder. Das Koppelelement 18 wird gebildet aus einem Vorsprung 70, der in den gewählten Darstellungen aus der Zeichenebene heraus hervorsteht, und einem Mitnehmer 72, an dem der Vorsprung 70 in Figur 15 anliegt. Da der Vorsprung 70 am Mitnehmer 72 anliegt, befindet sich das Koppelelement 18 in der Koppelstellung.

Das Freigabeelement 22 wird im gezeigten Ausführungsbeispiel durch ein Zahnrad 74 und ein Rastelement 76 gebildet. Das Rastelement 76 kann im gezeigten Ausführungsbeispiel nach oben und unten verschoben und somit mit dem Zahnrad 74 in Eingriff und außer Eingriff gebracht werden. Dies entspricht der Freigabestellung wenn das Rastelement 76 nicht in Eingriff mit dem Zahnrad 74 ist und der Sperrstellung, wenn der Eingriff vorliegt.

Figur 15 zeigt die Situation am Beginn eines Schrittes in der maximalen plantarflektierten Stellung. Der Fuß liegt also vollflächig auf dem Boden auf und das Fußteil 2 ist maximal gegen das Unterschenkelteil 4 gegen den Urzeigersinn verschwenkt. Der Winkel zwischen dem Unterschenkelteil 4 und dem Fußteil 2 ist maximal. Dadurch hat der Vorsprung 70, der drehfest mit dem Fußteil 2 verbunden ist, den Mitnehmer 72, der drehfest mit dem Zahnrad 74 verbunden ist, relativ zum Unterschenkelteil 4 gegen den Urzeigersinn verschwenkt und dabei die nicht dargestellte Feder, die den ersten Energiespeicher darstellt, gespannt und mit Energie aufgeladen. Die Zähne des Zahnrades 74 sind so ausgebildet, dass eine Verschwenkung des Zahnrades 74 um die Schwenkachse 6 in dieser Richtung möglich ist, da das Rastelement 76 auf den schrägen Zähnen des Zahnrades 74 entlanggleitet.

Die Figuren 16 und 17 zeigen die mittlere Standphase (Figur 16) und die terminale Standphase (Figur 17). Das Fußteil 2 wird gegenüber dem Unterschenkelteil 4 im Uhrzeigersinn verschwenkt, so dass es zu einer Dorsalflexion kommt. Das Freigabeelement 22 bleibt dabei in der Sperrstellung. Das Rastelement 76 befindet sich sowohl in Figur 16 als auch in Figur 17 in Eingriff mit den Zähnen des Zahnrades 74. Dadurch wird verhindert, dass sich das Zahnrad 74 um die Schwenkachse 6 im Urzeigersinn herum dreht, wodurch der erste Energiespeicher energetisch entladen würde. Da dies verhindert wird, befindet sich das Freigabeelement 22 in der Sperrstellung. Das Koppelelement 18 hingegen befindet sich in der Entkoppelstellung. Der Vorsprung 70 befindet sich weder in Figur 16 noch in Figur 17 in Kontakt mit dem Mitnehmer 72, so dass die Entkoppelstellung vorliegt. In Figur 17 befindet sich das Gelenk in der maximal dorsalflektierten Stellung. Der Winkel zwischen dem Fußteil 2 und dem Unterschenkelteil 4 ist minimal.

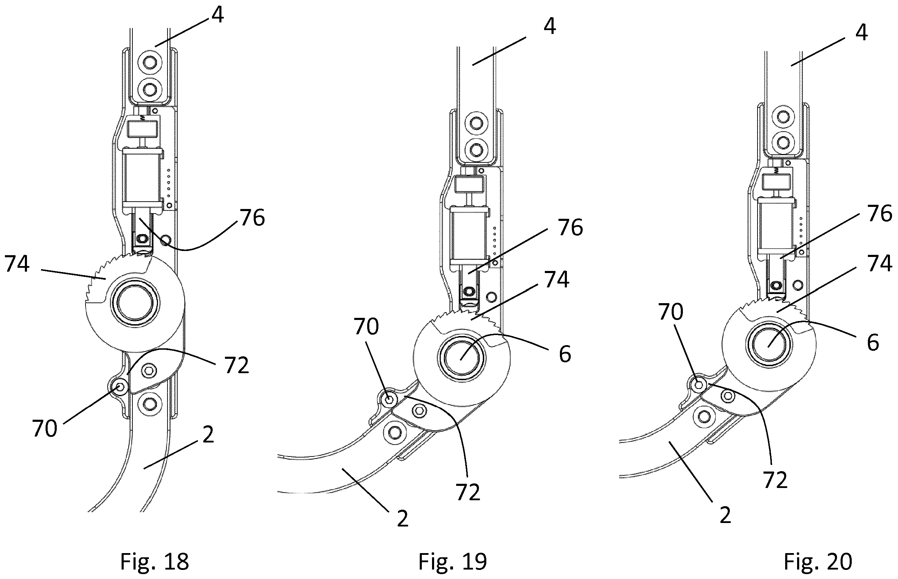

Im weiteren Verlauf des Schrittes kommt es erneut zu einer Plantarflexion wenn die Kraft vom Fuß auf den Träger der Orthese oder Prothese mit der Gelenkeinrichtung übertragen wird um sich in Richtung auf den nächsten Schritt vom Boden abzustoßen. Dies geschieht so lange bis die in Figur 18 dargestellte Situation erreicht ist. Sie entspricht der in Figur 15 gezeigten maximalplantar flektierten Stellung des Fußteils 2 relativ zum Unterschenkelteil 4. Das Koppelelement 18 befindet sich wieder in der Koppelstellung, da der Vorsprung 70 am Mitnehmer 72 anliegt. Das Freigabeelement 22 befindet sich weiterhin in der Sperrstellung, da das Rastelement 76 weiterhin in Eingriff mit dem Zahnrad 74 steht.

In der nachfolgenden Schwungphase, die in Figur 19 dargestellt ist, wird das Freigabeelement 22 aus der Sperrstellung in die Freigabestellung gebracht. Man erkennt, dass das Rastelement 76 nach oben verschoben wird und somit nicht mehr in Eingriff mit dem Zahnrad 74 steht. Dieses wird nun durch die im ersten Energiespeicher, der nicht dargestellt ist, gespeicherte mechanische Energie um die Schwenkachse 6 herum im Uhrzeigersinn verschwenkt. Da der Mitnehmer 72 drehfest mit diesem Zahnrad 74 verbunden ist dreht auch er sich im Uhrzeigersinn um die Schwenkachse und verschiebt damit den anliegenden Vorsprung 70 ebenfalls in diese Richtung. Das Fußteil 2 wird aufgrund dieser Energiefreisetzung durch den ersten Energiespeicher relativ zum Unterschenkelteil 4 im Uhrzeigersinn, also in Dorsalflexionsrichtung verschwenkt. Dies geschieht, da sich das Koppelelement 18 in der Koppelstellung befindet. Am Ende der Schwungphase, deren Situation in Figur 20 dargestellt ist, ist die Energie des ersten Energiespeichers vollständig abgegeben und das Fußteil 2 relativ zum Unterschenkelteil 4 verschwenkt worden. Zur Vorbereitung des nächsten Schrittes, der mit einem "Heelstrike" beginnt, wird das Freigabeelement 22 wieder in die Sperrstellung gebracht, indem das Rastelement 76 nach unten verschoben und wieder in Eingriff mit dem Zahnrad 74 gebracht wird. Das Koppelelement 18 befindet sich weiterhin in der Koppelstellung, da der Vorsprung 70 am Mitnehmer 72 anliegt. Beim nachfolgenden Fersenauftritt und Abrollen des Fußes kommt es zu einer Plantarflexion, also im gezeigten Ausführungsbeispiel einer Verschwenkung des Fußteils 2 relativ zum Unterschenkelteil 4 um die Schwenkachse 6 entgegen dem Uhrzeigersinn, bis die in Figur 15 dargestellte Position erreicht wird.

### Bezugszeichenliste:

- 2: Fußteil
- 4: Unterschenkelteil
- 6: Schwenkachse
- 8: erster Energiespeicher
- 10: Zylinder
- 12: Kolben
- 14: Feder
- 16: Stößel
- 18: Koppelelement
- 20: Ferse
- 22: Freigabeelement
- 24: Sperrklinke
- 26: zweiter Energiespeicher
- 28: Pfeil
- 30: Volumen
- 32: Fluidleitung
- 34: schaltbares Ventil
- 36: Ausgleichsreservoir
- 38: Druckspeicher
- 40: zweites schaltbares Ventil
- 42: Volumen
- 44: Rotationshydraulik
- 46: elektronische Datenverarbeitungseinrichtung
- 48: Spannungsversorgung
- 50: elektrische Leitung
- 52: Steuerleitung
- 54: Sensorik
- 56: Gelenk
- 58: Oberschenkelschale
- 60: Unterschenkelschale
- 62: Fersenauftritt
- 64: maximum Plantarflexion
- 66: maximum Dorsalflexion
- 68: Abheben
- 70: Vorsprung
- 72: Mitnehmer
- 74: Zahnrad
- 76: Rastelement

## Patentansprüche

1. Orthopädietechnische Gelenkeinrichtung mit
(a) einem Unterschenkelteil (4),
(b) einem Fußteil (2), das um eine Schwenkachse (6) schwenkbar an dem Unterschenkelteil angeordnet ist,
(c) wenigstens einem ersten Energiespeicher (8) und
(d) einem Koppelelement (18), das in eine Koppelstellung bringbar ist, in der ein Verschwenken des Fußteils relativ zu dem Unterschenkelteil um die Schwenkachse in einer Plantarflexionsrichtung zu einer Erhöhung der in dem ersten Energiespeicher gespeicherten Energiemenge führt,
**dadurch gekennzeichnet, dass** das Koppelelement in eine Entkoppelstellung bringbar ist, und
die orthopädietechnische Gelenkeinrichtung wenigstens ein Freigabeelement (22) aufweist, das
in eine Freigabestellung und
in eine Sperrstellung bringbar ist, wobei
die in dem ersten Energiespeicher gespeicherte Energiemenge freigebbar ist, indem das Freigabeelement in die Freigabestellung gebracht wird.

2. Orthopädietechnische Gelenkeinrichtung nach Anspruch 1, **dadurch gekennzeichnet, dass** die in dem ersten Energiespeicher gespeicherte Energiemenge durch ein Verschwenken des Fußteils relativ zum Unterschenkelteil nicht beeinflussbar ist, wenn sich das Koppelelement in der Entkoppelstellung befindet.

3. Orthopädietechnische Gelenkeinrichtung nach einem der vorstehenden Ansprüche, **dadurch gekennzeichnet, dass** die orthopädietechnische Gelenkeinrichtung eine elektrische Steuerung aufweist, die eingerichtet ist, das wenigstens eine Freigabeelement aus der Sperrstellung in die Freigabestellung zu bringen und umgekehrt.

4. Orthopädietechnische Gelenkeinrichtung nach Anspruch 3, **dadurch gekennzeichnet, dass** die orthopädietechnische Gelenkeinrichtung wenigstens einen Sensor (54), insbesondere wenigstens einen Druck-, Lage-, Kraft- oder Beschleunigungssensor aufweist, der eingerichtet ist wenigstens einen Messwert zu erfassen und an die elektrische Steuerung zu übermitteln.

5. Orthopädietechnische Gelenkeinrichtung nach Anspruch 4, **dadurch gekennzeichnet, dass** die elektrische Steuerung eingerichtet ist, das wenigstens eine Freigabeelement, in Abhängigkeit von dem wenigstens einen übermittelten Messwert, aus der Sperrstellung in die Freigabestellung zu bringen.

6. Orthopädietechnische Gelenkeinrichtung nach einem der Ansprüche 4 oder 5, **dadurch gekennzeichnet, dass** die elektrische Steuerung eingerichtet ist, anhand des zumindest einen übermittelten Messwerts einen Verlust eines Bodenkontakts der orthopädietechnischen Gelenkeinrichtung zu detektieren, und das Freigabeelement aus der Sperrstellung in die Freigabestellung zu bringen, wenn der Verlust des Bodenkontakts detektiert wurde.

7. Orthopädietechnische Gelenkeinrichtung nach einem der Ansprüche 3 bis 6, **dadurch gekennzeichnet, dass** die elektrische Steuerung eingerichtet ist, das Koppelelement erst dann von der Koppelstellung in die Entkoppelstellung zu bringen, wenn sich das Freigabeelement in der Sperrstellung befindet.

8. Orthopädietechnische Gelenkeinrichtung nach einem der vorstehenden Ansprüche, **dadurch gekennzeichnet, dass** die orthopädietechnische Gelenkeinrichtung wenigstens einen zweiten Energiespeicher (26) aufweist, der derart eingerichtet ist, dass ein Verschwenken des Fußteils relativ zum Unterschenkelteil um die Schwenkachse in einer Dorsalflexionsrichtung zu einer Erhöhung der in dem zweiten Energiespeicher gespeicherten Energiemenge führt.

9. Orthopädietechnische Gelenkeinrichtung nach einem der vorstehenden Ansprüche, **dadurch gekennzeichnet, dass** es sich bei dem wenigstens einen ersten Energiespeicher und/oder dem wenigstens einen zweiten Energiespeicher um ein Federelement und/oder eine hydraulische und/oder eine pneumatische Vorrichtung handelt.

10. Orthopädietechnische Gelenkeinrichtung nach einem der vorstehenden Ansprüche, **dadurch gekennzeichnet, dass** das Koppelelement als Fluid-Ventil-Kombination ausgebildet ist.

11. Orthopädietechnische Gelenkeinrichtung Anspruch 9 und 10, **dadurch gekennzeichnet, dass** der Gelenkeinrichtung zwei Rotationshydrauliken zugeordnet sind.

## Claims

1. Orthopedic joint device with
(a) a lower leg part (4),
(b) a foot part (2) that is arranged on the lower leg part about a swivel axis such that it can be swivelled,
(c) at least a first energy store (8), and
(d) a coupling element (18) that can be brought into a coupling position, in which a swivelling of the foot part relative to the lower leg part about the swivel axis in a plantar flexion direction leads to an increase in the amount of energy stored in the first energy store, **characterized by the fact that**
the coupling element can be brought into a de-coupling position, and
the orthopedic joint device has at least one release element (22), which can be brought into
a release position and
a locked position, wherein
the energy stored in the first energy store can be released by bringing the release element into the release position.

2. The orthopedic joint device according to claim 1, **characterized by the fact that** the energy stored in the first energy store cannot be influenced by a swivelling of the foot part relative to the lower leg part when the coupling element is in the de-coupling position.

3. The orthopedic joint device according to one of the above claims, **characterized by the fact that** the orthopedic joint device comprises an electric control system that is configured to bring the at least one release element out of the locked position and into the release position, and vice-versa.

4. The orthopedic joint device according to claim 3, **characterized by the fact that** the orthopedic joint device features at least one sensor (54), particularly at least one pressure, position, force or acceleration sensor, which is configured to record at least one measured value and to transmit it to the electric control system.

5. The orthopedic joint device according to claim 4, **characterized by the fact that** the electric control system is configured to bring the at least one release element out of the locked position into the release position, depending on the at least one transmitted measure value.

6. The orthopedic joint device according to one of the claims 4 or 5, **characterized by the fact that** the electric control system is configured to detect a loss of ground contact of the orthopedic joint device using the at least one transmitted measured value, and to bring the release element out of the locked position into the release position when the loss of ground contact has been detected.

7. The orthopedic joint device according to one of the claims 3 to 6, **characterized by the fact that** the electric control system is configured only to bring the coupling element from the coupling position into the de-coupling position when the release element is in the locked position.

8. The orthopedic joint device according to one of the above claims, **characterized by the fact that** the the orthopedic joint device comprises at least a second energy store (26), which is configured in such a way that a swivelling of the foot part relative to the lower leg part about the swivel axis in a dorsal flexion direction leads to an increase in the amount of energy stored in the second energy store.

9. The orthopedic joint device according to one of the above claims, **characterized by the fact that** the at least first energy store and/or the at least second energy store preferably refer(s) to a spring element and/or a hydraulic and/or a pneumatic device.

10. The orthopedic joint device according to one of the above claims, **characterized by the fact that** the coupling element is designed as a fluid-valve combination.

11. The orthopedic joint device according to claims 9 and 10, **characterized by the fact that** two rotary hydraulics are allocated to the joint device.

## Revendications

1. Dispositif d'articulation orthopédique comprenant
(a) une partie bas de jambe (4),
(b) une partie pied (2), qui est disposée sur la partie bas de jambe de manière à pouvoir pivoter autour d'un axe de pivotement (6),
(c) au moins un premier accumulateur d'énergie (8), et
(d) un élément de couplage (18) qui peut être amené dans une position de couplage, dans laquelle un pivotement de la partie pied par rapport à la partie bas de jambe autour de l'axe de pivotement dans une direction de flexion plantaire provoque une augmentation de la quantité d'énergie stockée dans le premier accumulateur d'énergie,
**caractérisé en ce que**
l'élément de couplage peut être amené dans une position de découplage, et
le dispositif d'articulation orthopédique présente au moins un élément de libération (22) qui peut être amené
dans une position de libération et
dans une position de blocage, sachant que
la quantité d'énergie stockée dans le premier accumulateur d'énergie peut être libérée du fait que l'élément de libération est amené dans la position de libération.

2. Dispositif d'articulation orthopédique selon la revendication 1, **caractérisé en ce que** la quantité d'énergie stockée dans le premier accumulateur d'énergie ne peut pas être influencée par un pivotement de la partie pied par rapport à la partie bas de jambe lorsque l'élément de couplage se trouve dans la position de découplage.

3. Dispositif d'articulation orthopédique selon l'une des revendications précédentes,
**caractérisé en ce que** le dispositif d'articulation orthopédique comporte une commande électrique qui est conçue pour amener ledit au moins un élément de libération de la position de blocage jusque dans la position de libération, et inversement.

4. Dispositif d'articulation orthopédique selon la revendication 3, **caractérisé en ce que** le dispositif d'articulation orthopédique comporte au moins un capteur (54), en particulier au moins un capteur de pression, de position, de force ou d'accélération, qui est conçu pour détecter au moins une valeur de mesure et pour la transmettre à la commande électrique.

5. Dispositif d'articulation orthopédique selon la revendication 4, **caractérisé en ce que** la commande électrique est conçue pour amener ledit au moins un élément de libération de la position de blocage jusque dans la position de libération en fonction de ladite au moins une valeur de mesure transmise.

6. Dispositif d'articulation orthopédique selon l'une des revendications 4 ou 5,
**caractérisé en ce que** la commande électrique est conçue pour détecter une perte de contact avec le sol du dispositif d'articulation orthopédique à l'aide de ladite au moins une valeur de mesure transmise, et pour amener l'élément de libération de la position de blocage jusque dans la position de libération lorsque la perte de contact avec le sol a été détectée.

7. Dispositif d'articulation orthopédique selon l'une des revendications 3 à 6,
**caractérisé en ce que** la commande électrique est conçue pour amener l'élément de couplage de la position de couplage jusque dans la position de découplage uniquement lorsque l'élément de libération se trouve dans la position de blocage.

8. Dispositif d'articulation orthopédique selon l'une des revendications précédentes,
**caractérisé en ce que** le dispositif d'articulation orthopédique comporte au moins un deuxième accumulateur d'énergie (26) qui est conçu de telle sorte qu'un pivotement de la partie pied par rapport à la partie bas de jambe autour de l'axe de pivotement dans une direction de flexion dorsale provoque une augmentation de la quantité d'énergie stockée dans le deuxième accumulateur d'énergie.

9. Dispositif d'articulation orthopédique selon l'une des revendications précédentes,
**caractérisé en ce que** ledit au moins un premier accumulateur d'énergie et/ou ledit au moins un deuxième accumulateur d'énergie est un élément de ressort et/ou un dispositif hydraulique et/ou un dispositif pneumatique.

10. Dispositif d'articulation orthopédique selon l'une des revendications précédentes,
**caractérisé en ce que** l'élément de couplage est réalisé sous forme de combinaison fluide-valve.

11. Dispositif d'articulation orthopédique selon les revendications 9 et 10, **caractérisé en ce que** deux systèmes hydrauliques rotatifs sont associés au dispositif d'articulation.
